(19) Europäisches Patentamt
European Patent Office
Office européen des brevets

(11) **EP 3 607 878 A1**

(12) **EUROPEAN PATENT APPLICATION**

(43) Date of publication:
**12.02.2020  Bulletin 2020/07**

(51) Int Cl.:
*A61B 5/024* (2006.01)    *A61B 5/0456* (2006.01)
*A61B 5/00* (2006.01)    *A61B 5/04* (2006.01)
*A61B 8/02* (2006.01)    *A61B 8/08* (2006.01)

(21) Application number: **18187431.4**

(22) Date of filing: **06.08.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AE Eindhoven (NL)**

(72) Inventors:
• **ZHANG, Ying**
  **5656 AE Eindhoven (NL)**
• **JIN, Sheng**
  **5656 AE Eindhoven (NL)**
• **YIN, Bin**
  **5656 AE Eindhoven (NL)**

(74) Representative: **de Haan, Poul Erik et al**
  **Philips International B.V.**
  **Philips Intellectual Property & Standards**
  **High Tech Campus 5**
  **5656 AE Eindhoven (NL)**

(54) **CARDIOTOCOGRAPHIC SIGNALS CLASSIFICATION**

(57) In a cardiotocographic categorization method, an initial fetal heart rate (FHR) baseline is determined for a cardiotocographic window. The initial FHR baseline is refined for the cardiotocographic window using an iterative refinement process that includes: iteratively identifying FHR accelerations and decelerations in the cardiotocographic window using the FHR baseline; and determining a refined FHR baseline for the cardiotocographic window with the identified FHR accelerations and decelerations excluded. The initial fetal heart rate baseline is replaced with the refined fetal heart rate baseline to repeat the iterative refinement process until a predefined validation criterion is met. A category is assigned for the cardiotocographic window using a cardiotocographic categorization guideline operating on the FHR baseline and the FHR accelerations and decelerations identified in the last repetition of the iterative refinement process. In some embodiments, the FHR baseline determined is based on characteristics of a histogram of FHR values in the cardiotocographic window.

*Fig. 1*

EP 3 607 878 A1

## Description

FIELD OF THE INVENTION

The following relates generally to the fetal monitoring arts, cardiotocography (CTG) arts, home fetal monitoring arts, and related arts.

BACKGROUND OF THE INVENTION

Cardiotocography (CTG) provides dual monitoring of the fetal heart rate (FHR) and uterine contractions during pregnancy and labor. This combination of information is particularly useful to assess fetal wellbeing during delivery, but is also used earlier in the pregnancy, especially the fetal heart rate monitoring aspect. An electronic fetal monitor (EFM) is commonly used to record CTG data. The EFM includes a heart rate monitor, commonly implemented as a Doppler ultrasound heart rate monitor employing an ultrasound transducer place on the mother's abdomen, and a tocometer which measures uterine contractions, commonly implemented as a pressure transducer placed at the fundus of the uterus, i.e. "below" the ultrasound transducer. The EFM may also detect other vital signs or information, such as automatically detecting fetal movements in the Doppler ultrasound data, providing a hand control via which the mother manually indicates fetal movements felt by the mother, and/or so forth.

Interpretation of the CTG data is complex. Commonly extracted vital signs include the baseline fetal heart rate (sometimes concisely referred to herein as "baseline"), fetal heart rate variability, the number of accelerations of the fetal heart rate, and likewise the number of decelerations of the fetal heart rate. In practical clinical settings, diagnostic conclusions are sometimes derived by qualitative visual inspection of the CTG traces reliant upon the clinician's experience. This can lead to interobserver variability, and even intraobserver variability. One study reports that when four obstetricians examined 50 cardiotocograms, they agreed in only 22% of cases; and, two months later, the same clinicians interpreted 21% of the same tracings in a different way from what they did the first time. Fanelli et al. "Quantitative Assessment of Fetal Well-Being through CTG Recordings: A New Parameter Based on Phase-Rectified Signal Average", IEEE Journal of Biomedical and Health Informatics, vol. 17, no. 5 (September 2013). Moreover, visual inspection is unable to detect more complex events that can be extracted only by using quantitative approaches that mine information content of fetal heart rate variability.

To alleviate these problems, it is known to employ a standardized scoring algorithm to analyze the CTG signal to assess fetal distress according to fetal heart rate baseline, fetal heart rate variability, the number of accelerations and the number of decelerations. Based on the analysis result, categorization of fetal heart rate patterns is done according to a guideline. One example is the three-tier CTG categorization guideline described in Macones et al., "The 2008 National Institute of Child Health and Human Development workshop report on electronic fetal monitoring: update on definitions, interpretation, and research guidelines", Journal of Obstetric, Gynecologic, & Neonatal Nursing 37.5 (2008), pages 510-515 (hereinafter "NICHD 2008"). This guideline, or variants thereof, are employed in some clinical settings.

A brief summary of metrics and the scoring system promulgated the NICHD 2008 guideline is as follows. The baseline fetal heart rate is determined by approximating the mean fetal heart rate during a 10-minute window, excluding accelerations and decelerations and periods of marked fetal heart rate variability (>25 bpm). There must be at least two minutes of identifiable baseline segments (not necessarily contiguous) in any 10-minute window, or the baseline for that period is indeterminate. Baseline fetal heart rate variability (in beats per minute, bpm) is determined in a 10-minute window, excluding accelerations and decelerations, and is scored as absent, minimal ($\leq$ 5 bpm), moderate (6-25 bpm), or marked (>25 bpm). Moderate fetal heart rate variability is considered indicative of positive fetal wellbeing. Accelerations constitute abrupt increases of $\geq$ 15 bpm in fetal heart rate lasting for $\geq$ 15 sec, with the increase from onset to peak in $\leq$ 30 sec. Different standards apply before 32 weeks of gestation. Classification of decelerations is more complex and defines different types of deceleration attributable to different mechanisms. The NICHD 2008 guideline categorizes the fetal heart rate pattern using the baseline fetal heart rate, fetal heart rate variability, number of accelerations, and number of decelerations as one of: Category I (normal), Category III (abnormal), and Category II (indeterminate).

The CTG signal is advantageously analyzed according to a guideline such as the NICHD 2008 guideline or some variant thereof, rather than based on subjective visual assessment of the CTG traces, so as to avoid inter- and intra-observer variation. Appropriate CTG interpretation can lead to timely intervention in the case of fetal distress, or conversely can deter unnecessary intervention that could be detrimental to the fetus or mother. CTG scoring is also useful in home monitoring settings where clinician assessment of the CTG is not available.

The following discloses certain improvements.

SUMMARY OF THE INVENTION

In some embodiments disclosed herein, a method of classifying a cardiotocographic signal is disclosed. An initial fetal heart rate baseline is estimated for a cardiotocographic window representing the cardiotocographic signal within a time window. The initial fetal heart rate baseline for the cardiotocographic window is refined by using an iterative refinement process including: identifying fetal heart rate accelerations and decelerations in the cardiotocographic window based on the initial fetal heart rate baseline; determining a refined fetal heart rate baseline for the cardiotocographic window by excluding the identified fetal heart rate accelerations and decelerations from the cardiotocographic window; and replacing the initial fetal heart rate baseline with the refined fetal heart rate baseline to repeat the iterative refinement process until a predefined validation criterion is met. One of a plurality of predefined categories is assigned to the cardiotocographic window using a cardiotocographic categorization guideline operating on the fetal heart rate baseline and the fetal heart rate accelerations and decelerations identified in the last repetition of the iterative refinement process.

In some embodiments disclosed herein, a nontransitory computer readable medium has stored thereon program code readable and executable by one or more electronic processors to perform a method of classifying a cardiotocographic signal as set forth in the immediately preceding paragraph.

In some embodiments disclosed herein, a device for classifying a cardiotocographic signal is disclosed, comprising a display, an electronic processor operatively connected to control the display, and a nontransitory computer readable medium having stored thereon program code readable and executable by the electronic processor to perform a method of classifying the cardiotocographic signal. In this method, a FHR baseline is determined for a cardiotocographic window representing the cardiotocographic signal within a time window using a histogram-based method. The histogram-based method includes computing a histogram of FHR values in the cardiotocographic window, classifying the histogram with respect to a number of dominant bins, and setting the FHR baseline to a FHR value of the single dominant bin if the histogram is classified as having a single dominant bin, or to an average of the FHR values of the dominant bins if the histogram is classified as having more than one dominant bin. One of a plurality of predefined categories is assigned to the cardiotocographic window using a cardiotocographic categorization guideline operating on the FHR baseline and on FHR accelerations and decelerations identified respective to the FHR baseline.

One advantage resides in providing more accurate guideline categorization for a CTG.

Another advantage resides in deriving a more accurate fetal heart rate (FHR) baseline for a CTG.

Another advantage resides in providing more accurate identification and characterization of FHR accelerations and decelerations in a CTG.

Another advantage resides in providing one or more of the foregoing benefits in conjunction with improved processing speed.

A given embodiment may provide none, one, two, more, or all of the foregoing advantages, and/or may provide other advantages as will become apparent to one of ordinary skill in the art upon reading and understanding the present disclosure.

BRIEF DESCRIPTION OF THE DRAWINGS

The invention may take form in various components and arrangements of components, and in various steps and arrangements of steps. The drawings are only for purposes of illustrating the preferred embodiments and are not to be construed as limiting the invention.

FIGURE 1 diagrammatically illustrates an electronic fetal monitor (EFM) configured to perform CTG categorization according to one embodiment.
FIGURE 2 diagrammatically illustrates CTG categorization suitably implemented by an EFM according to another embodiment.
FIGURE 3 diagrammatically illustrates a process for fetal heart rate baseline determination and identification of fetal heart rate accelerations and decelerations according to another embodiment.
FIGURE 4 diagrammatically illustrates CTG data (top plot) and a corresponding histogram (bottom plot) for a histogram classified as Type 1.
FIGURE 5 diagrammatically illustrates CTG data (top plot) and a corresponding histogram (bottom plot) for a histogram classified as Type 2.
FIGURE 6 diagrammatically illustrates validation/labeling of FHR accelerations according to another embodiment.
FIGURE 7 diagrammatically illustrates validation/labeling of FHR decelerations according to another embodiment.
FIGURE 8 diagrammatically illustrates some suitable features that can be calculated for a fetal heart rate acceleration candidate (left side) and for a fetal heart rate deceleration candidate (right side) according to another embodiment.

DETAILED DESCRIPTION OF EMBODIMENTS

A difficulty with applying a CTG guideline such as the NICHD 2008 guideline is that the various parameters are interdependent. Accelerations and decelerations are defined with respect to the fetal heart rate (FHR) baseline, as they are FHR excursions above or below that baseline, respectively. But, the FHR baseline is in turn defined in terms of the FHR accelerations and decelerations insofar as the FHR baseline is determined by (in part) excluding the FHR accelerations and decelerations from the baseline analysis.

In one aspect disclosed herein, the fetal heart rate baseline and the accelerations and decelerations are identified using an iterative process. By iteratively refining the FHR baseline and the FHR accelerations and decelerations together, their interrelated impact on each other can be better accommodated.

In another disclosed aspect, a histogram-based method for determining the FHR baseline is disclosed. Compared with generating the FHR baseline by averaging over the time window, the histogram-based method advantageously limits the impact of FHR accelerations and decelerations on the generated FHR baseline. This is because these FHR excursions are usually a small portion of the total time window, and hence do not contribute to the dominant one or two bins of the histogram. Averaging over two dominant bins in the histogram-based method also improves accuracy by accommodating cases in which the actual FHR baseline is near a boundary between two bins resulting in two dominant bins each accumulating FHR samples with values near that boundary. While the histogram-based method for determining the FHR baseline has certain advantages such as those mentioned above, other approaches for determining the FHR baseline which do not employ a histogram may be employed, such as averaging the FHR samples over the time window.

In some contemplated embodiments, the aspect of employing an iterative refinement process that adjusts a FHR baseline estimate and the FHR accelerations and decelerations is performed alone. In some contemplated embodiments, the aspect of using the histogram-based method for FHR baseline estimation is performed alone. In the illustrative embodiments, both aspects are combined, e.g. in the iterative refinement process the FHR baseline estimate is determined for the time window using the histogram-based method, and/or the baseline is re-determined for the time window using the histogram-based method but now excluding the identified FHR accelerations and decelerations.

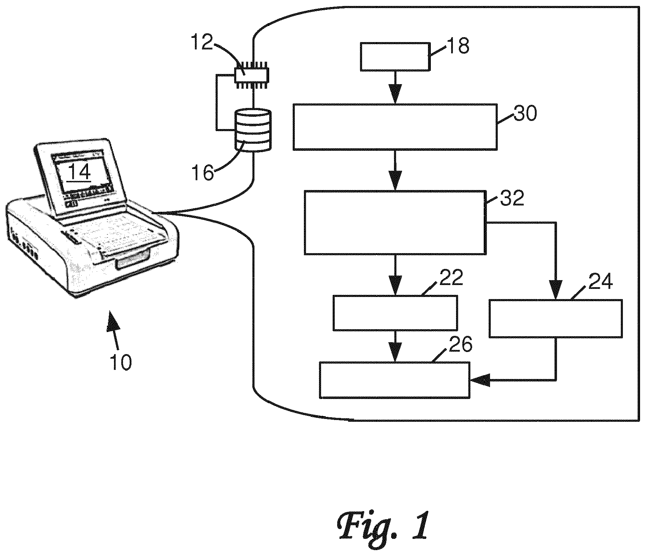

With reference now to FIGURE 1, an illustrative electronic fetal monitor (EFM) 10 configured to perform CTG categorization. The illustrative EFM may, for example, be a Philips Avalon EFM (available from Koninklijke Philips N.V., Eindhoven, the Netherlands) employing the Doppler ultrasound modality to monitor FHR and a pressure transducer to acquired tocometer data. Other types of EFM devices, optionally employing different FHR and/or toco monitoring modalities, may alternatively be employed. The EFM 10 includes or is operatively connected with (e.g. via a wired or wireless connection) an ultrasound transducer and tocometer pressure transducer (components not shown) for performing the ultrasound FHR monitoring and tocometry, respectively. The EFM 10 further includes an electronic processor 12 (internal component typically contained within the housing of the EFM 10 but diagrammatically indicated for illustrative purposes in FIGURE 1, suitably embodied for example as a microprocessor or microcontroller) and includes or is in operative connection with (e.g. via a wired or wireless connection) a display 14.

The EFM 10 further includes or is in operative connection with (e.g. via a wired or wireless connection) a non-transitory storage medium 16, for example comprising a read only memory (ROM), programmable read only memory (PROM), CMOS memory, flash memory, various combinations thereof, or the like. The non-transitory storage medium 16 stores machine-readable instructions which are readable and executable by the electronic processor 12 to control and/or read the ultrasound, pressure transducer, or other CTG data acquisition modality or modalities to acquire CTG data and to perform the disclosed CTG categorization. The non-transitory storage medium 16 may further include random access memory (RAM), FLASH memory, and/or other fast read/write memory for storing the collected CTG data, e.g. so as to implement a buffer 18 storing CTG data acquired over a time window. The CTG data stored in the buffer 18 thus constitutes a cardiotocographic window representing the cardiotocographic signal within a time window. In general, the cardiotocographic signal includes two signal components: a fetal heart rate signal component, and a tocogram component. For the NICHD 2008 scoring guideline, the time window is suitably a 10 minute time window, although some other window size is alternatively contemplated.

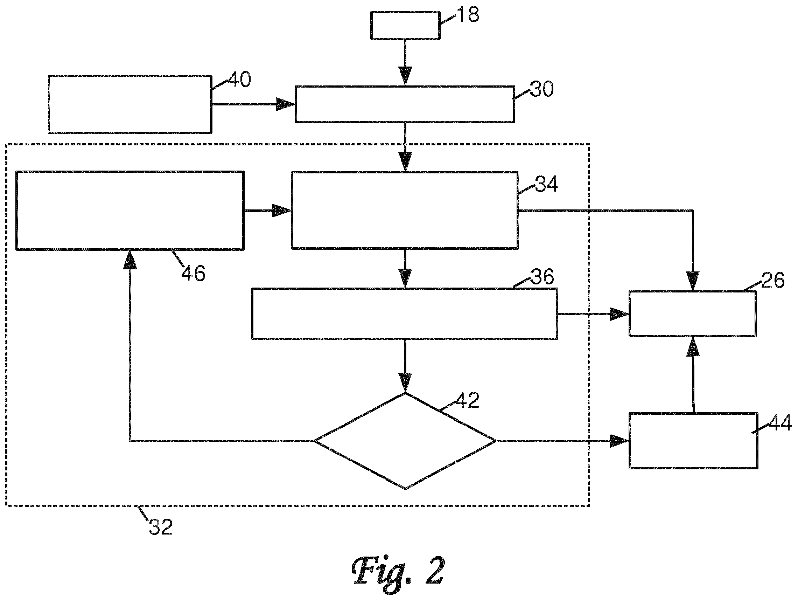

The CTG categorization implemented by the electronic processor 12 programmed by instructions stored on the non-transitory storage medium 16 to extract CTG parameters including at least the FHR baseline 22, FHR accelerations and decelerations 24, and for the NICHD 2008 guideline also including FHR variability (not indicated in FIGURE 1, but see FIGURE 2). A categorization process 26 operates on the FHR baseline 22, FHR accelerations and decelerations 24, and optionally on the FHR variability to assign one of a plurality of predefined categories to the cardiotocographic window using a cardiotocographic categorization guideline operating on the FHR baseline 22 and the fetal heart rate accelerations and decelerations 24 identified in the last repetition of the iterative refinement process. The categorization process 26 may, for example, employ the NICHD 2008 guideline described in Macones et al., "The 2008 National Institute of Child Health and Human Development workshop report on electronic fetal monitoring: update on definitions, interpretation, and research guidelines", Journal of Obstetric, Gynecologic, & Neonatal Nursing 37.5 (2008), pages 510-515. A human-perceptible representation of the category assigned to the cardiotocographic window is presented. For example the NICHD 2008 guideline identifies three possible fetal wellbeing categories: Normal; Indeterminate; or Abnormal. The presentation of the human-perceptible representation of the assigned category may, for example, be the category (Normal, Indeterminate, or Abnormal) displayed on the display 14. Additionally or alternatively, the human-perceptible representation of the assigned category may be a visual alarm built into the EFM 10, such as lighting a yellow LED if the assigned category is indeterminate, or a flashing red LED if the assigned category is determined to be Abnormal. Additionally or alternatively, the human-perceptible representation of the assigned category may be an audible alarm output by a loudspeaker built into the EFM 10, e.g. an audible alarm may be sounded if the assigned category of the cardiotocographic window is Abnormal. These are merely non-limiting illustrative examples.

With continuing reference to FIGURE 1, CTG parameters are extracted as follows. Histogram-based FHR baseline estimation **30** is performed to generate a FHR baseline estimate for the cardiotocographic window. The FHR baseline **22** for the cardiotocographic window is then determined by refining the FHR baseline estimate using an iterative refinement process **32** including iteratively: (i) performing FHR accelerations and decelerations identification **34** in the cardiotocographic window using the FHR baseline estimate, and (ii) re-determining **36** the FHR baseline estimate for the cardiotocographic window with the identified FHR accelerations and decelerations excluded.

With reference to FIGURE 2, the CTG categorization implemented by the EFM **10** of FIGURE 1 is described in further detail. The histogram-based FHR baseline estimation **30** is performed to determine the initial FHR baseline estimate. This initial estimate may be computed for the entire 10 min time window. Using the histogram-based approach for FHR baseline estimation disclosed herein reduced (but does not eliminate) error in the FHR baseline estimate due to accelerations and decelerations (which ideally should be excluded when estimating the baseline, but cannot be excluded in operation **30** since the accelerations and decelerations have not yet been identified). Alternatively, the initial FHR baseline estimate may be set to an FHR baseline **40** from a last CTG report if such is available. The iterative refinement process **32** then commences. FHR accelerations and decelerations identification **34** is performed in the cardiotocographic window using the FHR baseline estimate. The accelerations and decelerations are identified respective to the FHR baseline estimate from operation **30**. Since the FHR estimate of the operation **30** may have some error as noted above, this error can propagate to introduce some error in the identified accelerations and decelerations, although the identifications should be close. In operation **36**, the FHR baseline estimate is re-determined for the cardiotocographic window, preferably again using the histogram-based approach but now with the FHR accelerations and decelerations identified in operation **34** excluded. This is expected to provide a more accurate baseline estimate. In a validation operation **42**, the FHR baseline estimate from operation **30** is compared with the re-determined FHR baseline estimate from operation **36**. If these two baselines are sufficiently close (e.g., having a difference of 2 bpm or less in some embodiments, or having a difference of 1 bpm or less in some other embodiments) then the results are validated, and iterative refinement process **32** terminates with the FHR baseline and identified FHR accelerations and decelerations of the last iteration as outputs. Thereafter, in an operation **44** the FHR variability is determined. In the NICHD 2008 standard, the baseline FHR variability is determined as the amplitude of the peak-to-trough variations (excluding FHR accelerations and decelerations) in beats per minute, e.g. moderate FHR variability (considered to be most positively indicative of fetal wellbeing) is classified as variability of between 6 bpm and 25 bpm. The categorization process **26** then operates on the FHR baseline, FHR accelerations and decelerations, and FHR variability to assign a category for the cardiotocographic window, e.g. using the NICHD 2008 guideline.

On the other hand, if at the validation operation **42** it is determined that two baselines from respective operations **30**, **36** are not sufficiently close to be validated (e.g., having a difference of greater than 2 bpm in some embodiments, or having a difference of greater than 1 bpm in some other embodiments) then in an operation **46** the FHR baseline estimate is set to the re-determined FHR baseline estimate output by the operation **36**, and flow returns to operation **34** to identify the FHR accelerations and decelerations using this updated FHR baseline estimate. The iterative process **32** may thereby iterate as appropriate until at operation **42** validation is achieved, e.g. until the iteration-over-iteration change in FHR baseline is 2 bpm or less in some embodiments, or until iteration-over-iteration change in FHR baseline is 1 bpm or less in some other embodiments.

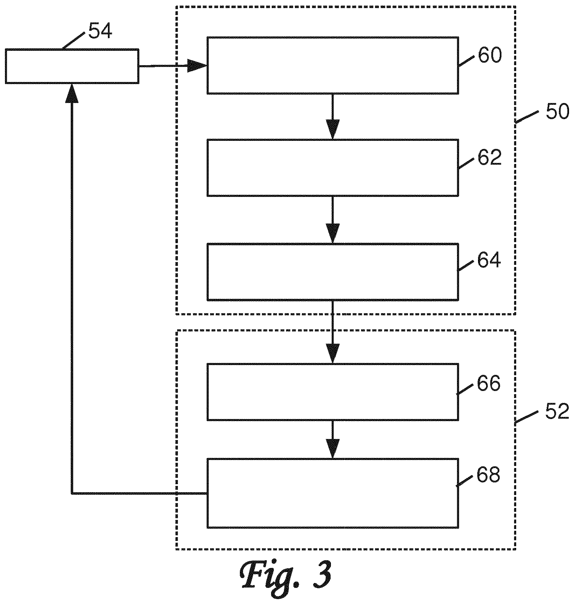

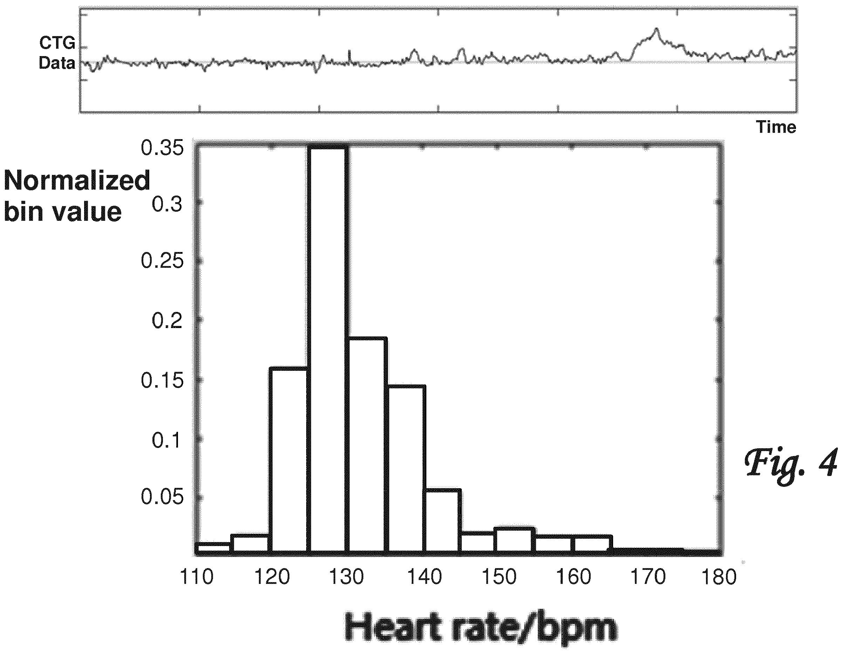

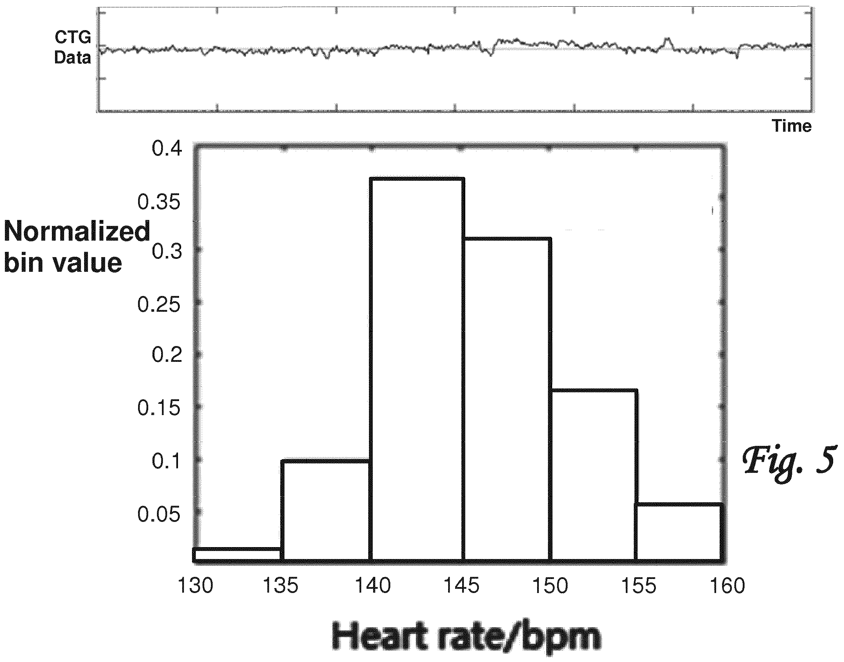

With reference now to FIGURES 3, 4, and 5, an illustrative histogram-based FHR baseline determination **50** in conjunction with a process **52** for identifying portions of the 10 min time window that are likely to be FHR accelerations or decelerations are described. Input CTG data **54** to the histogram-based FHR baseline determination **50** is the 10 min time window with any already-identified FHR accelerations or decelerations cut out. More particularly, with brief reference back to FIGURES 1 and 2, for the initial FHR baseline estimation **30** the input CTG data **54** is the entire 10 min time window since no FHR accelerations or decelerations have yet been identified; whereas, for the re-determination **36** of the FHR baseline estimate the input CTG data **54** is the 10 min time window with any FHR accelerations or decelerations identified in the immediately preceding iteration of the operation **34** cut out (i.e. removed). In an operation **60**, the CTG histogram for the CTG data **54** is generated. The histogram bin size is chosen based on the desired FHR baseline resolution and the available statistics (e.g. number of samples). In the illustrative examples of FIGURES 4 and 5, the bin size is 5 bpm, and the illustrative bins include bins for bpm intervals of: 110-115 bpm; 115-120 bpm; 120-125 bpm; 125-130 bpm; 130-135 bpm; and so on. The value stored in each bin may be the total number of FHR samples in the CTG data **54** that are in the bin bpm range, or a normalized version of this value (that is, normalized so that the sum of all bin values equals unity). FIGURES 4 and 5 illustrate two examples of CTG data (top plot) with corresponding histograms (bottom plot). In an operation **62** of FIGURE 3, the histogram is classified with respect to a number of dominant bins. In one classification scheme, the histogram may be classified into one of the following three classes: (1) the histogram has a single dominant bin (FIGURE 4 is an example); (2) the histogram has two dominant bins (FIGURE 5 is an example); or (3) the histogram has no dominant bins. This classification could be extended, e.g. to include a class for histograms with three dominant bins. The term "dominant bin" can be variously defined, e.g. for the illustrative classification scheme with one, two, or no dominant bins, a dominant bin may be suitably defined as any bin storing a normalized bin value of 0.3 or higher (i.e., a dominant bin is any bin that contains at least 30% of the CTG samples of the CTG data **54**); for such a scheme the likelihood of three dominant bins each containing at least 30% of the CTG samples is vanishingly small as such a set of three dominant bins would contain over 90% of the CTG samples. Using this classification scheme, the histogram for the CTG data of FIGURE 4 has a single dominant bin corresponding to the FHR interval of 125-130 bpm; while the CTG data of FIGURE 5 has two dominant bins corresponding to the FHR intervals of 140-145 bpm and 145-150 bpm.

As another example, if the classification is extended to encompass a class for histograms with three dominant bins then a dominant bin may be defined as having normalized bin value $\geq 0.22$, so that the likelihood of four dominant bins each containing at least 20% of the CTG samples is vanishingly small as the four dominant bins would contain over 88% of the samples.

In operation **64** of FIGURE 3, the FHR baseline estimate is determined based on the histogram and its classification. In the illustrative example, the FHR baseline estimate is determined as follows: (1) if the histogram is classified as having a single dominant bin (e.g. FIGURE 4) then the FHR baseline estimate is set to a FHR value of the single dominant bin; (2) if the histogram is classified as having two (or more, variant embodiments with a class for, e.g. three dominant bins) dominant bins then the FHR baseline estimate is set to an average FHR value of the two (or more) dominant bins; and if the histogram is classified as having no dominant bins then setting the FHR baseline estimate to an average FHR value over the cardiotocographic window. Using this estimation process, the histogram of the CTG data of FIGURE 4 has a single dominant bin corresponding to the FHR interval of 125-130 bpm and so the FHR baseline for the CTG data of FIGURE 4 is suitably set to 125 bpm. (Alternatively, it could be set to the middle FHR value of the dominant bin, e.g. 127.5 bpm, but with bin size of 5 bpm this value is not reflective of the realistic bpm resolution). The histogram of the CTG data of FIGURE 5 is classified as having two dominant bins corresponding to the FHR intervals of 140-145 bpm and 145-150 bpm, so that an average FHR value of these two bins is used. In one approach, the resulting FHR estimate is (140+145)/2=142.5 bpm. In a variant embodiment, a weighted average FHR value of the two bins is used, where the bins are weighted by their values (i.e. sample counts). In the histogram of FIGURE 5, the bin for the FHR interval of 140-145 bpm has a value of 0.37 while the bin for the FHR interval of 145-150 bpm has a value of 0.31 so that the FHR baseline estimate is:

$$\frac{0.37 * 140 + 0.31 * 145}{0.37 + 0.31} = 142 \text{ bpm}$$

In this example, the weighting did not modify the FHR baseline estimate by much; however, the weighting can have more effect if one dominant bin is much larger than the other dominant bin. As used herein, averaging of the FHR values of the two (or more) dominant bins is to be understood as encompassing either a straight average or a weighted average, e.g. weighted by the normalized bin values as in the last example above.

With continuing reference to FIGURE 3, the process **52** for identifying portions of the 10 min time window that are likely to be FHR accelerations or decelerations (*cf.* FHR acc/dec identification operation **34** of FIGURES 1 and 2) includes an operation **66** in which the CTG data **54** is searched on a point-by-point basis to detect accelerations or decelerations. In an operation **68** the identified accelerations and/or decelerations are excluded from the CTG data **54** and the new CTG data set (with those accelerations and/or decelerations cut out) serves as the CTG data input to the histogram-based FHR baseline estimation performed to implement the re-determination **36** of the FHR baseline.

A suitable implementation of the point-by-point search **66** is as follows. Baseline signal segments are searched point by point based on FHR baseline estimate from the histogram-based estimation **50**. Put one segment of signal into a window with same size, and check if the segment satisfy a criterion (e.g. range of signal and distance between mean of the segment and the FHR baseline). If the criterion is satisfied of signal, extend the segment and continue the above process until the criterion is not satisfied. For the case that the final length of checked segment is over threshold, percentage of the ascending/descending segments will be calculated and exclude those segments with too many ascending/descending segments.

For the NICHD 2008, the FHR accelerations and decelerations are validated and/or labeled in specific ways. Suitable acc/dec validation/labeling approaches are described next.

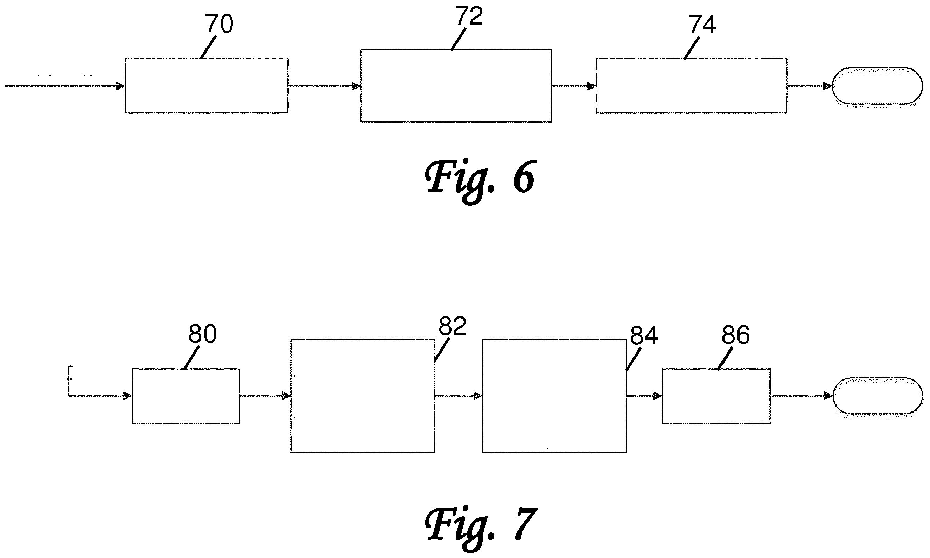

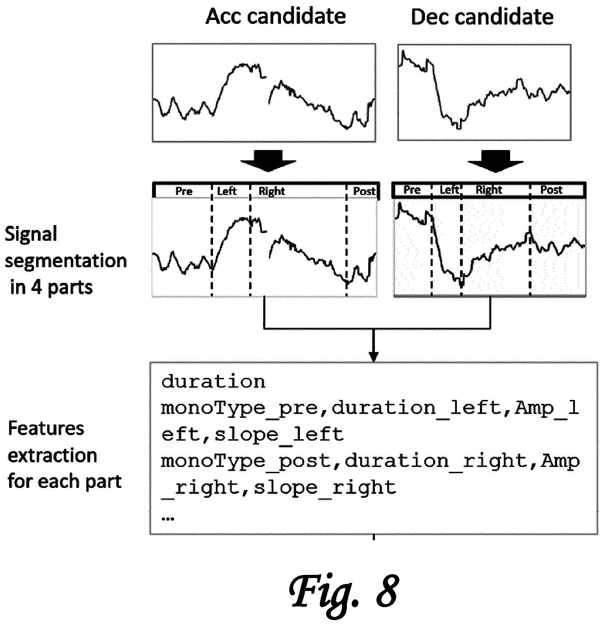

With reference to FIGURE 6, validation/labeling of FHR accelerations is illustrated. In an operation **70**, an acceleration candidate is identified. For example, a possible acceleration segment is calculated according to the criterion (for example, find the points over certain value, e.g. 15 bpm, from the FHR baseline and find left edge and right edge of the peak). At the beginning of the signal, points are checked whose distance from the FHR baseline is over 15 bpm, which is regarded as part of possible acceleration. Then points are searched forward and backward until the points return to near baseline. In an operation **72**, features of the acceleration candidate are calculated. FIGURE 8, left-hand side, illustrates some suitable features that can be calculated for the acceleration candidate. As shown in FIGURE 8, the acceleration candidate is separated into four parts: pre, left, right and post part. From each part, features are extracted such as duration, amplitude drop, monotony and slope of each part. Using these features, an operation **74** classifies the FHR acceleration candidate as a true acceleration.

With reference to FIGURE 7, the processing is similar for a FHR deceleration. In an operation **80**, a deceleration candidate is identified. For example, a possible deceleration segment is calculated according to the criterion (for example, find the points below a certain value, e.g. 15 bpm, from the FHR baseline and find left edge and right edge of the valley). At the beginning of the signal, points are checked whose distance from the FHR baseline is more than 15 bpm, which is regarded as part of possible deceleration. Then points are searched forward and backward until the points return to near baseline. In an operation **82**, features of the deceleration candidate are calculated. FIGURE 8, right-hand side, illustrates some suitable features that can be calculated for the deceleration candidate. As shown in FIGURE 8, the deceleration candidate is separated into four parts: pre, left, right and post part. From each part, features are extracted such as duration, amplitude drop, monotony and slope of each part. The approach for deceleration (FIGURE 7) differs from that for acceleration (FIGURE 6) in that the Toco signal is used to assess the deceleration type. In an operation **84**, features of the Toco signal are calculated. The features may suitably include a baseline value and metrics of peaks of the Toco signal. Based on this information, an amplitude drop of each Toco peak and time gap with the deceleration candidate can be derived. Using the FHR and Toco features derived in respective operations **82**, **84**, an operation **86** classifies the FHR deceleration candidate as a true deceleration and assigns an appropriate type (e.g., early deceleration, late deceleration, variable deceleration), for example according to the NICHD 2008 guideline or a variant thereof.

In the illustrative embodiments, the cardiotocographic window represents the cardiotocographic signal within a time window of an illustrative 10 minute length. In one approach, this is done by performing 10 minutes of cardiotocographic data collection and then stopping the data collection, estimating and refining the fetal heart rate baseline, and assigning one of the plurality of predefined categories to the cardiotocographic window as disclosed herein. In another approach, a sliding window is employed, with the classification being assigned once 10 minutes of cardiotocographic data is collected and thereafter cardiotocographic data collection continues with the assigned classification being updated at selected intervals, e.g. every minute in one non-limiting example, with each update using the last 10 minutes of collected cardiotocographic data. It is contemplated for such updating to be performed more frequently, for example at sufficiently short selected intervals so as to provide real-time assignment of the cardiotocographic classification.

The invention has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims or the equivalents thereof.

**Claims**

1. A method of classifying a cardiotocographic signal, the method comprising:

   estimating (30) an initial fetal heart rate baseline for a cardiotocographic window representing the cardiotocographic signal within a time window; refining the initial fetal heart rate baseline (22) for the cardiotocographic window by using an iterative refinement process (32) including:

   identifying (34) fetal heart rate accelerations and decelerations in the cardiotocographic window based on the initial fetal heart rate baseline; determining (36) a refined fetal heart rate baseline for the cardiotocographic window by excluding the identified fetal heart rate accelerations and decelerations from the cardiotocographic window; and replacing (46) the initial fetal heart rate baseline with the refined fetal heart rate baseline to repeat the iterative refinement process (32) until a predefined validation criterion is met; and

   assigning (26) one of a plurality of predefined categories to the cardiotocographic window using a cardiotocographic categorization guideline operating on the fetal heart rate baseline and the fetal heart rate accelerations and decelerations identified in the last repetition of the iterative refinement process.

2. The method of claim 1 further comprising presenting a human-perceptible representation of the category assigned to the cardiotocographic window.

3. The method of any one of claims 1-2 wherein the determining or re-determining (50) of the fetal heart rate baseline estimate is based on characteristics of a histogram of fetal heart rate values in the cardiotocographic window.

4. The method of claim 3 wherein the estimating (30) of the initial fetal heart rate baseline or the determining (36) of the refined fetal heart rate baseline includes:

classifying (62) the histogram with respect to a number of dominant bins;

if the histogram is classified as having a single dominant bin then setting the fetal heart rate baseline to a fetal heart rate value of the single dominant bin;

if the histogram is classified as having two or more dominant bins then setting the fetal heart rate baseline to an average fetal heart rate value of the two or more dominant bins; and

if the histogram is classified as having no dominant bins then setting the fetal heart rate baseline to an average fetal heart rate value over the cardiotocographic window.

5. The method of claim 4 wherein the classifying (62) comprises classifying the histogram as to whether the histogram has a single dominant bin, two dominant bins, or no dominant bins, and if the histogram is classified as having two dominant bins then setting the fetal heart rate baseline to an average fetal heart rate value of the two dominant bins.

6. The method of any one of claims 1-2 wherein the estimating (30) of the initial fetal heart rate baseline comprises setting the initial fetal heart rate baseline equal to a fetal heart rate baseline (40) of a previous cardiotocographic window.

7. The method of any one of claims 1-6 wherein identifying (34) fetal heart rate accelerations and decelerations in the cardiotocographic window using the initial fetal heart rate baseline includes:

identifying candidate fetal heart rate accelerations and decelerations (70, 80) based on distance of the fetal heart rate from the initial fetal heart rate baseline;

calculating (72, 82) features of the candidate fetal heart rate accelerations and decelerations; and

identifying (74, 86) fetal heart rate accelerations and decelerations as candidate fetal heart rate accelerations and decelerations that satisfy a selection criterion based at least on the features of the candidate fetal heart rate accelerations and decelerations.

8. The method of claim 7 wherein identifying (86) fetal heart rate decelerations in the cardiotocographic window further includes:

calculating (84) features of a tocogram component of the cardiotocographic window;

wherein the fetal heart rate decelerations are identified (86) as candidate fetal heart rate decelerations that satisfy the selection criterion based on the features of the candidate fetal heart rate decelerations and further based on the features of the tocogram component of the cardiotocographic window.

9. A non-transitory computer readable medium (16) having stored thereon program code readable and executable by one or more electronic processors (12) to perform a method of classifying a cardiotocographic signal as set forth in any one of claims 1-8.

10. A device for classifying a cardiotocographic signal, the device comprising:

a display (14);

an electronic processor (12) operatively connected to control the display; and

a non-transitory computer readable medium (16) having stored thereon program code readable and executable by the electronic processor to perform a method of classifying the cardiotocographic signal, the method including:

determining a fetal heart rate baseline (22) for a cardiotocographic window representing the cardiotocographic signal within a time window using a histogram-based method (50) including computing (60) a histogram of fetal heart rate values in the cardiotocographic window, classifying (62) the histogram with respect to a number of dominant bins, and setting (64) the fetal heart rate baseline to a fetal heart rate value of the single dominant bin if the histogram is classified as having a single dominant bin, or to an average of the fetal heart rate values of the dominant bins if the histogram is classified as having more than one dominant bin; and

assigning (26) a one of a plurality of predefined categories to the cardiotocographic window using a cardiotocographic categorization guideline operating on the fetal heart rate baseline and on fetal heart rate accelerations and decelerations identified (34) respective to the fetal heart rate baseline.

11. The device of claim 10 wherein the method further includes controlling the display to present a human-perceptible representation of the category assigned to the cardiotocographic window.

12. The device of any one of claims 10-11 wherein the classifying (62) of the histogram comprises classifying the histogram as to whether the histogram has a single dominant bin, two dominant bins, or no dominant bins, and if the histogram is classified as having two dominant bins then setting the fetal heart rate baseline to an average fetal heart rate value of the two dominant bins.

13. The device of claim 12 wherein the determining of the fetal heart rate baseline (22) further includes:

setting the fetal heart rate baseline to an average fetal heart rate value over the cardiotocographic window if the histogram is classified as having no dominant bins.

14. The device of any one of claims 10-13 wherein determining the fetal heart rate baseline (22) includes:

estimating (30) an initial fetal heart rate baseline for the cardiotocographic window using the histogram-based method (50); and determining a refined fetal heart rate baseline (22) for the cardiotocographic window and fetal heart rate accelerations and decelerations (24) in the cardiotocographic window using an iterative refinement process (32) that both adjusts the initial fetal heart rate baseline and determines the fetal heart rate accelerations and decelerations in each iteration of the iterative refinement process.

15. The device of claim 14 wherein the iterative refinement process (32) includes, in each iteration:

identifying (34) fetal heart rate accelerations and decelerations in the cardiotocographic window respective to the initial fetal heart rate baseline; determining (36) a refined fetal heart rate baseline for the cardiotocographic window using the histogram-based method (50) with the identified fetal heart rate accelerations and decelerations excluded when computing the histogram of fetal heart rate values in the cardiotocographic window; and replacing (46) the initial fetal heart rate baseline with the refined fetal heart rate baseline to repeat the iterative refinement process (32) until a predefined validation criterion is met.

Fig. 1

Fig. 2

Fig. 3

*Fig. 4*

*Fig. 5*

*Fig. 6*

*Fig. 7*

Fig. 8

EP 3 607 878 A1

Europäisches
Patentamt

European
Patent Office

Office européen
des brevets

# EUROPEAN SEARCH REPORT

Application Number

EP 18 18 7431

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/133115 A1 (HAMILTON EMILY F [CA] ET AL) 8 July 2004 (2004-07-08)<br>* paragraphs [0002], [0005], [0048], [0049], [0061], [0064], [0066], [0075] *<br>* paragraphs [0130], [0134], [0136], [0176], [0190], [0194] *<br>* paragraphs [0226], [0227], [0244], [0247], [0250], [0254] *<br>* figure 29A * | 1,2,4,5, 7-15 | INV.<br>A61B5/024<br>A61B5/0456<br>A61B5/00<br>A61B5/04<br>A61B8/02<br>A61B8/08 |
| X | WO 2014/029986 A1 (FERNANDES & CO GLOBAL HEALTHCARE SOLUTIONS LTD [GB]) 27 February 2014 (2014-02-27)<br>* page 10, lines 29, 30 *<br>* page 11, lines 6, 7 *<br>* page 16, line 13 *<br>* figures 1, 7 * | 1,2,6-9 | |
| X | MANTEL R ET AL: "Computer analysis of antepartum fetal heart rate: 1. Baseline determination",<br>INTERNATIONAL JOURNAL OF BIO-MEDICAL COMPUTING, ELSEVIER SCIENCE PUBLISHERS, SHANNON, IE,<br>vol. 25, no. 4, 1 May 1990 (1990-05-01), pages 261-272, XP023157528,<br>ISSN: 0020-7101, DOI: 10.1016/0020-7101(90)90030-X<br>[retrieved on 1990-05-01]<br>* page 263, lines 27-29 *<br>* page 264, lines 16-36 * | 1,3,9 | TECHNICAL FIELDS SEARCHED (IPC)<br><br>A61B |

-/--

The present search report has been drawn up for all claims

| Place of search | Date of completion of the search | Examiner |
|---|---|---|
| The Hague | 14 January 2019 | Meyer, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 18 18 7431

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | G. S. DAWES ET AL: "Baseline in human fetal heart-rate records", BJOG: AN INTERNATIONAL JOURNAL OF OBSTETRICS AND GYNAECOLOGY, vol. 89, no. 4, 1 April 1982 (1982-04-01), pages 270-275, XP055541393, GB ISSN: 1470-0328, DOI: 10.1111/j.1471-0528.1982.tb04695.x * page 270, right-hand column, lines 12-15 * * figures 1-4 * | 10 | |
| A | MANTEL R ET AL: "Computer analysis of antepartum fetal heart rate: 2. Detection of accelerations and decelerations", INTERNATIONAL JOURNAL OF BIO-MEDICAL COMPUTING, ELSEVIER SCIENCE PUBLISHERS, SHANNON, IE, vol. 25, no. 4, 1 May 1990 (1990-05-01), pages 273-286, XP023157529, ISSN: 0020-7101, DOI: 10.1016/0020-7101(90)90031-0 [retrieved on 1990-05-01] * the whole document * | 1-15 | TECHNICAL FIELDS SEARCHED (IPC) |

The present search report has been drawn up for all claims

| Place of search | Date of completion of the search | Examiner |
|---|---|---|
| The Hague | 14 January 2019 | Meyer, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 18 7431

This annex lists the patent family members relating to the patent documents cited in the above-mentioned European search report.
The members are as contained in the European Patent Office EDP file on
The European Patent Office is in no way liable for these particulars which are merely given for the purpose of information.

14-01-2019

| Patent document cited in search report | Publication date | Patent family member(s) | Publication date |
|---|---|---|---|
| US 2004133115 A1 | 08-07-2004 | CA      2447861 A1<br>US  2004133115 A1 | 01-05-2004<br>08-07-2004 |
| WO 2014029986 A1 | 27-02-2014 | NONE | |

EPO FORM P0459

For more details about this annex : see Official Journal of the European Patent Office, No. 12/82

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Non-patent literature cited in the description

- **FANELLI et al.** Quantitative Assessment of Fetal Well-Being through CTG Recordings: A New Parameter Based on Phase-Rectified Signal Average. *IEEE Journal of Biomedical and Health Informatics,* September 2013, vol. 17 (5

- **MACONES et al.** The 2008 National Institute of Child Health and Human Development workshop report on electronic fetal monitoring: update on definitions, interpretation, and research guidelines. *Journal of Obstetric, Gynecologic, & Neonatal Nursing,* 2008, vol. 37.5, 510-515